# EUROPÄISCHE PATENTSCHRIFT

(11) **EP 2 119 441 B1**
(45) Veröffentlichungstag und Bekanntmachung des Hinweises auf die Patenterteilung: **20.05.2015**
(21) Anmeldenummer: 09160568.3
(22) Anmeldetag: 24.07.2004
(51) Int. Cl.: A61K 31/416, A61K 31/4184, A61K 45/06, A61P 3/10, A61P 5/50, A61P 9/12

(54) **Verwendung von Angiotensin II Rezeptor Antagonisten zur Steigerung der Insulinsensitivität**
Use of angiotensin II receptor antagonists in order to increase insulin sensitivity
Utilisation d'antagonistes du récepteur de l'angiotensine II pour augmenter la sensibilité à l'insuline

(30) Priorität: 31.07.2003 DE 10335027; 06.10.2003 DE 10346260; 05.12.2003 DE 10356815
(43) Veröffentlichungstag der Anmeldung: 18.11.2009
(62) Teilanmeldung aus: 04763484.5
(73) Patentinhaber: BOEHRINGER INGELHEIM INTERNATIONAL GMBH, 55216 Ingelheim am Rhein (DE); Boehringer Ingelheim Pharma GmbH & Co. KG, 55216 Ingelheim (DE)
(72) Erfinder: Unger, Thomas, 14195 Berlin (DE); Kintscher, Ulrich, 10437 Berlin (DE); Schupp, Michael, Philadelphia PA 19103 (US); Kauschke, Stefan, 88400 Biberach (DE); Mark, Michael, 88400 Biberach (DE)
(74) Vertreter: Hoffmann Eitle

(56) Entgegenhaltungen:
- WO-A-01/30353
- WO-A-96/40258
- WO-A-2004/014308
- WO-A-2004/062557
- WO-A-2004/062729
- WO-A-2004/071385
- WO-A1-00/27397
- WO-A1-99/65500
- WO-A2-00/02543
- WO-A2-01/76573
- SHARMA ARYA M ET AL: "Angiotensin blockade prevents type 2 diabetes by formation of fat cells." HYPERTENSION. NOV 2002, Bd. 40, Nr. 5, November 2002 (2002-11), Seiten 609-611, XP001182230 ISSN: 1524-4563
- ASMAR R ET AL: "Effects of telmisartan on arterial compliance and endothelial function in type 2 diabetes patients with essential hypertension" AMERICAN JOURNAL OF HYPERTENSION, Bd. 14, Nr. 4 Part 2, April 2001 (2001-04), Seite 114A, XP001182229 & SIXTEENTH ANNUAL SCIENTIFIC MEETING OF THE AMERICAN SOCIETY OF HYPERTENSION; SAN FRANCISCO, CALIFORNIA, USA; MAY 15-19, 2001 ISSN: 0895-7061
- PERSHADSINGH H A ET AL: "Insulin-Sensitizing Effects of Telmisartan: Implications for treating insulin-resistant hypertension and cardiovascular disease [1]" DIABETES CARE 2004 UNITED STATES, Bd. 27, Nr. 4, 2004, Seite 1015, XP009032922 ISSN: 0149-5992
- BENSON STEPHEN C ET AL: "Identification of telmisartan as a unique angiotensin II receptor antagonist with selective PPARgamma-modulating activity." HYPERTENSION. MAY 2004, Bd. 43, Nr. 5, Mai 2004 (2004-05), Seiten 993-1002, XP009032921 ISSN: 1524-4563
- SCHUPP MICHAEL ET AL: "Angiotensin type 1 receptor blockers induce peroxisome proliferator-activated receptor-gamma activity." CIRCULATION. 4 MAY 2004, Bd. 109, Nr. 17, 4. Mai 2004 (2004-05-04), Seiten 2054-2057, XP009032920 ISSN: 1524-4539
- DATABASE BIOSIS [Online] BIOSCIENCES INFORMATION SERVICE, PHILADELPHIA, PA, US Dezember 2000 STANGIER JOACHIM ET AL: 'Pharmacokinetics of repeated oral doses of amlodipine and amlodipine plus telmisartan in healthy volunteers' Database accession no. PREV200100018529 & STANGIER JOACHIM ET AL: "Pharmacokinetics of repeated oral doses of amlodipine and amlodipine plus telmisartan in healthy volunteers", JOURNAL OF CLINICAL PHARMACOLOGY, vol. 40, no. 12 Part 1, December 2000 (2000-12), pages 1347-1354, ISSN: 0091-2700
- DATABASE EMBASE [Online] ELSEVIER SCIENCE PUBLISHERS, AMSTERDAM, NL 1999 NEUTEL J M ET AL: 'The efficacy and safety of telmisartan compared to enalapril in patients with severe hypertension' Database accession no. EMB-1999153594 & NEUTEL J M ET AL: "The efficacy and safety of telmisartan compared to enalapril in patients with severe hypertension", INTERNATIONAL JOURNAL OF CLINICAL PRACTICE 1999 GB, vol. 53, no. 3, 1999, pages 175-178, ISSN: 1368-5031
- DATABASE MEDLINE [Online] US NATIONAL LIBRARY OF MEDICINE (NLM), BETHESDA, MD, US März 2002 GOKHALE NITIN ET AL: 'Efficacy and safety of losartan-amplodipine combination--an Indian postmarketing surveillance experience.' Database accession no. NLM12408291 & GOKHALE NITIN ET AL: "Efficacy and safety of losartan-amplodipine combination--an Indian postmarketing surveillance experience.", JOURNAL OF THE INDIAN MEDICAL ASSOCIATION MAR 2002 LNKD- PUBMED:12408291, vol. 100, no. 3, March 2002 (2002-03), pages 207-208, ISSN: 0019-5847
- DATABASE EMBASE [Online] ELSEVIER SCIENCE PUBLISHERS, AMSTERDAM, NL 15 Mai 2005 VITALE C ET AL: 'Metabolic effect of telmisartan and losartan in hypertensive patients with metabolic syndrome' Database accession no. EMB-2005468598 & VITALE C ET AL: "Metabolic effect of telmisartan and losartan in hypertensive patients with metabolic syndrome", CARDIOVASCULAR DIABETOLOGY 20050515 GB LNKD- DOI:10.1186/1475-2840-4-6, vol. 4, 15 May 2005 (2005-05-15), ISSN: 1475-2840
- MEIGS JAMES B ET AL: "The natural history of progression from normal glucose tolerance to type 2 diabetes in the Baltimore Longitudinal Study of Aging.", DIABETES, vol. 52, no. 6, June 2003 (2003-06), pages 1475-1484, ISSN: 0012-1797
- Shoba Rao: "Impaired Glucose Tolerance and Impaired Fasting Glucose", AMERICAN FAMILY PHYSICIAN, vol. 69, no. 8, 15 April 2004 (2004-04-15) , pages 1961-1968, XP055103317,
- DAVIDSON MAYER B ET AL: "Revisiting the oral glucose tolerance test criterion for the diagnosis of diabetes", JOURNAL OF GENERAL INTERNAL MEDICINE, PHILADELPHIA, PA, US, vol. 15, no. 8, 1 August 2000 (2000-08-01) , pages 551-555, XP002501454, ISSN: 0884-8734, DOI: 10.1046/J.1525-1497.2000.08024.X
- Anonymous: "Follow-up Report on the Diagnosis of Diabetes Mellitus", Clinical Diabetes, vol. 22, no. 2, 1 April 2004 (2004-04-01), pages 3160-3167, XP055103330, ISSN: 0891-8929, DOI: 10.2337/diaclin.22.2.71

## Beschreibung

Die Erfindung fällt in das Gebiet der Angiotensin II Rezeptor Antagonisten und betrifft die Verwendung der Angiotensin II Rezeptor Antagonisten Irbesarten und Telmisartan zur Behandlung von Personen bei denen Diabetes Mellitus Typ 2 diagnostiziert wurde oder Verdacht auf Prädiabetes besteht oder zur Prävention von Diabetes.

Diabetes mellitus Typ 2 ist die Manifestation zweier pathophysiologischer Phänomene, nämlich einer verminderten Insulinsekretion der Beta Zellen des Pankreas und einer Insulinresistenz der Zielorgane Leber, Skelettmuskulatur und Fettgewebe. In der Regel liegt eine komplexe Störung beider Komponenten vor. Die Erkrankung wird als Nüchtern-Hyperglykämie diagnostiziert, d.h. die Blutzuckerkonzentration nach 10-12 Stunden Fasten liegt über dem Grenzwert von 125 mg Glucose pro dl Plasma. Eine gezielte Behandlung des manifesten Typ 2 Diabetes ist durch Verbindungen der Substanzklasse der Thiazolidinedione (Glitazone) möglich. Diese Verbindungen verbessern die Nutzung des zirkulierenden Insulins und führen so zu einer Absenkung der Blutzuckerwerte (Insulinsensitizer). Gleichzeitig werden über Rückkoppelungsmechanismen die erhöhten Insulinspiegel reduziert und damit die Bauchspeicheldrüse entlastet. Insulinsensitizer wie Troglitazone, Rosiglitazone oder Pioglitazone entfalten diese Wirkung durch Bindung an bestimmte nukleäre Rezeptoren, PPAR-gamma genannt (Peroxisomal Proliferator Activated Receptor). Diese wirken als Transkriptionsregulatoren einer Reihe von Genen, die für den Glukose- und Lipidstoffwechsel von Bedeutung sind. Über diese Funktion können PPAR-gamma Liganden wie Prostaglandine oder die synthetischen Thiazolidinedione (Glitazone) zur Behandlung von Typ 2 Diabetes beitragen. Einer der Hauptmechanismen der Glukosesenkung durch PPAR-gamma Liganden ist die Induktion der Differenzierung von Adipocyten. Verstärkte Adipocytendifferenzierung und Remodelling des Fettgewebes hervorgerufen durch PPAR-gamma Liganden führt zu einer Umleitung bzw. Umverteilung freier Fettsäuren von der Skelettmuskulatur ins Fettgewebe und erhöht dadurch den Glukose Metabolismus in den Muskeln.

Da zum Zeitpunkt der Diagnose z.B. jeder zweite Typ-2-Diabetes Patient Zeichen einer koronaren Herzerkrankung aufweist, werden die Ursachen von Diabetes zunehmend in einer komplexen Stoffwechselstörung vermutet, die durch eine Reihe von Risikofaktoren wie gestörte Glukose-Toleranz, erhöhten Nüchternblutzucker, Insulinresistenz, Bluthochdruck, Dyslipidämie, oder Stammfettsucht angezeigt werden kann. Besonders ausgeprägt ist die Prävalenz einer Insulinresistenz bei Patienten mit Hypertriglyzeridämie und niedrigem HDL-Cholesterin. Man spricht vom Prä-Typ 2-Diabetes, Metabolischen Syndrom, Syndrom X, oder Insulin-Resistenz Syndrom. In einer ersten Phase bedingt eine verminderte Insulinantwort der Zielorgane eine Steigerung der pankreatischen Insulinsekretion, um den Blutzuckerspiegel im Normbereich zu halten. Nach mehreren Jahren überhöhter bzw. steigender Insulinproduktion kommt es dazu, dass die Insulinausschüttung durch die Beta-Zellen des Pankreas nicht weiter gesteigert werden kann. Damit beginnt die Phase der gestörten Glukosetoleranz. Der Organismus kann Glukose-Spitzenwerte nicht mehr rasch genug auffangen. Bleibt der Nüchternblutzucker schliesslich anhaltend hoch wird der Diabetes manifest.

WO 95/06410 offenbart die Verwendung von Angiotensin II Rezeptor Antagonisten zur Behandlung chronischer Entzündungserkrankungen einschließlich systemischer Autoimmunerkrankungen. Als eines von mehreren Beispielen für systemische Autoimmunerkrankungen wird Diabetes genannt. Den Autoimmunerkrankungen zugeordnet wird der Typ 1 Diabetes mellitus, der meist bei Jugendlichen unter 30 Jahren auftritt, wobei es bei entsprechender genetischer Disposition unter dem Einfluss verschiedener Faktoren zu einer Insulitis mit nachfolgender Zerstörung der B-Zellen kommt, sodass der Pankreas nur mehr wenig oder kein Insulin produzieren kann. Typ 2 Diabetes mellitus wird nicht als Autoimmunerkrankung angesehen.

Angiotensin II Rezeptor Antagonisten werden verwendet zur Behandlung von Bluthochdruck sowie von Folgeschäden kardiovaskulärer Endorgane, die mit Bluthochdruck in Verbindung gebracht werden. In der Fachliteratur werden sie in der Regel als metabolisch neutral eingestuft. Über eine Verbesserung der Insulin-Sensitivität im Tiermodell durch den Wirkstoff Irbesartan berichten Henriksen et al (Hypertension 38:884-90, 2001).

Sharma et al stellen in Hypertension 40(5): 609-611, 2001, die Hypothese auf, dass eine Blockade des Renin-Angiotensin Systems die Rekrutierung von Präadipozyten fördert wodurch die Anzahl kleiner insulin-sensitiver Adipozyten erhöht würde. Umverteilung von Lipiden aus Muskeln und anderem Gewebe ins Fettgewebe würde dann in einer verbesserten Insulin-Resistenz resultieren.

Asmar et al berichten im American Journal of Hypertension 14(4): 114A, 2001, dass Telmisartan in hypertensiven Patienten mit Typ 2 Diabetes die arterielle Steifigkeit vermindert.

Ziel der vorliegenden Erfindung ist es, Arzneimittel bereitzustellen, die sowohl zur Behandlung eines manifesten Typ 2 Diabetes als auch zur Behandlung erster Anzeichen der komplexen Stoffwechselstörung des Prädiabetes und damit zur Prävention eines Typ 2 Diabetes mellitus verwendet werden können. Im Rahmen der vorliegenden Erfindung wurde nun überraschend gefunden, dass die Angiotensin II Rezeptor Antagonisten Irbesartan und Telmisartan und ihre Salze neben der bekannten, den Blutdruck senkenden Wirkung, in einem zellulären System auch die Expression von Genen zu steigern vermögen, von deren Transkription bekannt ist, dass sie durch den PPARgamma Rezeptor reguliert wird. Dies eröffnet neue therapeutische Möglichkeiten bei der Behandlung und Prävention von Typ 2 Diabetes , Metabolischem Syndrom und Insulin Resistenz. Um vergleichbare Bedingungen zu gewährleisten wird dieser Effekt im Rahmen der vorliegenden Erfindung mit Hilfe einer stabil transormierten Zelllinie (vgl. Beispiel 2) beobachtet und quantifiziert. Dabei handelt es sich um CHO-Zellen, die das Ergebnis einer Transformation mit zwei Genkonstrukten darstellen. Das erste dieser Konstrukte kodiert für das Luciferase Gen aus *Photinus pyralis* (de Wet JR, Mol Cell Biol (1987) 7:725) unter der Kontrolle eines synthetischen Promotors mit einer fünffachen Wiederholung einer Hefe Gal4-Bindungsstelle (vgl. Genbank-Sequenz AF058756). Das zweite Konstrukt kodiert für ein Fusionsprotein bestehend aus der Ligandenbindungsdomäne des humanen PPARgamma2 Transkriprionsfaktors (vgl Genbank-Sequenz U79012) sowie der Hefe GAL4 DNA Bindungsdomäne (Aminosäuren 1-147; Sadowski I, Nucleic Acids Res (1989) 17:7539).

Die Induktion der Transkription von PPARgamma regulierten Genen ist von den als Antidiabetika verwendeten Thiazolidinedionen (z.B. Rosiglitazone) bekannt und wird durch deren Bindung an den PPARgamma Rezeptor und dessen Aktivierung bewirkt. Im Rahmen des hier angewandten Testsystems kann diese Wirkung als induzierte Luciferase Aktivität der transformierten Zellinie quantifiziert werden. Dieselbe Induktion einer Luciferase Aktivität erfolgt bei den Angiotensin II Rezeptor Antagonisten wider Erwarten nicht durch Bindung des Wirkstoffs an den Rezeptor PPARgamma. Die Induktion ist für den Wirkstoff Telmisartan besonders ausgeprägt. Eine Bindung von z.B. Telmisartan an den PPARgamma Rezeptor kann in verschiedenen Testsystemen nicht nachgewiesen werden. Es wird daher vermutet, dass die Erhöhung der Affinität von Cofaktorproteinen für PPARgamma durch einen Angiotensin II Rezeptor Antagonisten wie Telmisartan auch dann zur Rekrutierung der Cofaktorproteine führt, wenn hochaffine synthetische PPARgamma-Liganden nicht vorliegen. Dies bewirkt dann eine durch diese Cofaktoren vermittelte Aktivierung der Transkription von Genen, die durch den PPARgamma Rezeptor reguliert werden. Da nun die Induktion dieser Gene für die anti-diabetische Wirkung der Thiazolidinedione verantwortlich ist, kann davon ausgegangen werden, dass die Induktion derselben Gene durch Angiotensin II Rezeptor Antagonisten wie Telmisartan eine vergleichbare anti-diabetische Wirkung entfaltet. Somit eignen sich diese Wirkstoffe nicht nur zur Behandlung von Bluthochdruck sondern auch zur Behandlung und Prävention von Typ 2 Diabetes mellitus.

Die Entdeckung dieser neuen therapeutischen Wirkung der Angiotensin II Rezeptor Antagonisten Irbesartan und Telmisartan und ihrer Salze bedeutet, dass sie verwendet werden können zur Herstellung eines Arzneimittels für die Behandlung von Personen bei denen Diabetes Mellitus Typ 2 diagnostiziert wurde oder Verdacht auf Prädiabetes besteht, oder zur Prävention von Diabetes. Sie sind vor allem zur Behandlung und Prävention von Typ2-Diabetes und Prä-Typ2-Diabetes geeignet. Darin eingeschlossen ist die Behandlung und Prävention von Metabolischem Syndrom, Syndrom X, bzw. Insulin-Resistenz Syndrom. Wenn im Rahmen dieser Erfindung von zu behandelnden Personen die Rede ist, steht zwar primär die Vorstellung der Behandlung von bzw. der Prävention bei Menschen im Vordergrund, die eingesetzten Wirkstoffe und Wirkstoffkombinationen können aber auch in der Veterinärmedizin bei Säugetieren entsprechend eingesetzt werden.

Diabetes mellitus Typ 2 manifestiert sich in einem Nüchternblutzuckerwert, der 125 mg Glucose pro dl Plasma überschreitet, wobei die Bestimmung von Glucosewerten im Blut ein Standardverfahren in der medizinischen Routineanalytik darstellt. Wird eine Glukose Toleranz Test durchgeführt so liegt 2 Stunden nach nüchterner Einnahme von 75 g Glucose der Blutzuckerwert eines Diabetikers über 200mg Glucose pro dl Plasma. Bei einem Glukose Toleranz Test werden der zu untersuchenden Person nach 10-12 stündigem Fasten 75 g Glucose oral verabreicht und der Blutzuckerwert wird unmittelbar vor Einnahme sowie 1 bzw. 2 Stunden nach Einnahme der Glucose ermittelt. Bei einem Gesunden liegt der Blutzuckerwert vor Einnahme zwischen 60 und 110 mg pro dl Plasma, eine Stunde nach Einnahme unter 200 mg pro dl und nach 2 Stunden unter 140 mg pro dl. Liegt der Wert nach 2 Stunden zwischen 140 und 200 mg so spricht man auch von einer gestörten Glukosetoleranz.

Ein besonders starkes Indiz für das Vorliegen eines Prädiabetes ist, wenn eine Insulinresistenz nachgewiesen werden kann. So kann es sein, dass eine Person zur Aufrechterhaltung der Glukosehomöostase die 2-3fache Menge an Insulin benötigt als eine andere, ohne dass dies eine direkte pathologischen Bedeutung hätte. Als die aussagekräftigste Methode zur Bestimmung der Insulinresistenz gilt der euglykämisch-hyperinsulinämische Clamp-Test. Das Verhältnis Insulin zu Glukose wird im Rahmen einer kombinierten Insulin-Glukose-Infusionstechnik bestimmt. Von einer Insulinresistenz spricht man, wenn die Glucoseaufnahme unterhalb der 25. Perzentile der untersuchten Hintergrundspopulation liegt (WHO Definition). Etwas weniger aufwendig als die Clamp-Untersuchung sind sogenannte Minimalmodelle bei denen während eines intravenösen Glukosetoleranz-Tests in zeitlich festgelegten Abständen Insulin- und Glukosekonzentrationen im Blut gemessen werden und hieraus die Insulinresistenz berechnet wird. Ein weiteres Bestimmungsverfahren ist das mathematische HOMA-Modell. Die Insulinresistenz wird über die Nüchtern-Plasma-Glukose und die Nüchtern-Insulin-Konzentration berechnet. Im Rahmen dieser Methode ist es nicht möglich zwischen hepatischer und peripherer Insulinresistenz zu unterscheiden. Für eine Bewertung der Insulinresistenz in der täglichen Praxis sind diese Verfahren wenig geeignet. Im klinischen Alltag werden zur Einschätzung der Insulinresistenz in der Regel andere Parameter herangezogen. Bevorzugt wird dafür z.B. die Triglyzerid-Konzentrationen des Patienten herangezogen werden, da erhöhte Triglyzerid-Spiegel signifikant mit dem Vorliegen einer Insulinresistenz korrelieren.
In der Praxis geht man etwas vereinfachend davon aus, dass Personen dann Insulin resistent sind, wenn sie mindestens 2 der folgenden Eigenschaften aufweisen:
1) Übergewicht oder Fettleibigkeit
2) Bluthochdruck
3) Dyslipidaemie (veränderter Gehalt des Blutes an Gesamtlipiden)
4) mindestens einen Verwandten ersten Grades, bei dem eine gestörte Glukosetoleranz oder Typ 2 Diabetes diagnostiziert wurde.
Übergewicht bedeutet hier, dass der Body Mass Index (BMI) zwischen 25 und 30 kg/m² beträgt, wobei der BMI den Quotienten des Körpergewichts in kg und des Quadrats der Körpergrösse in Metern darstellt. Bei Fettleibigkeit ist der BMI grösser als 30 kg/m².
Aus obiger Definition der Insulin Resistenz wird unmittelbar ersichtlich, dass für ihre Behandlung Blutdrucksenker dann geeignet und angezeigt sind, wenn beim Patienten u.a. die Eigenschaft Bluthochdruck festgestellt wird.
So kann eine Behandlung von Type 2 Diabetikern mit
Telmisartan gleichzeitig zur Behandlung oder unterstützenden Behandlung einer Dyslipidämie eingesetzt werden. Übliche Dosiserungen von Telmisartan bewirken eine signifikante Verminderung der Plasma Werte von LDL-Cholesterin, Gesamt Cholesterin und/oder Triglyceriden.

Da Insulin Resistenz als ein Zustand angesehen wird, der ein allmähliches Ansteigen des Blutdrucks bewirkt, kann seine Behandlung mit Telmisartan trotz normaler Blutdruckwerte, als eine Bluthochdruck Prävention angesehen werden.

Ein ähnliches Indiz für Prädiabetes ist, wenn die Bedingungen für das Metabolische Syndrom erfüllt sind, dessen Hauptmerkmal die Insulin Resistenz ist. Gemäss den ATP IHINCEP Richtlinien (Executive Summary of the Third Report of the National Cholesterol Education Program (NCEP) im Journal of the American Medical Association 285:2486-2497, 2001) spricht man vom Metablischen Syndrom, wenn ein Patient mindestens 3 der folgenden Eigenschaften aufweist:
1) Abdominale Fettleibigkeit, definiert als Taillienumfang >40 inches or 102 cm bei Männern und >35 inches or 94 cm bei Frauen
2) Triglyceridwerte >150 mg/dl
3) HDL-cholesterol Werte <40 mg/dl bei Männern
4) Bluthochdruck >130/>85 mm Hg
5) Nüchternblutzucker von >110 mg/dl
Aus dieser Definition des Metabolischen Syyndroms wird unmittelbar ersichtlich, dass für seine Behandlung Blutdrucksenker dann geeignet sind, wenn beim Patienten u.a. Bluthochdruck festgestellt wird.

Da auch das Metabolische Syndrom als ein Zustand angesehen wird, der ein allmähliches Ansteigen des Blutdrucks bewirkt, kann seine Behandlung mit Telmisartan trotz normaler Blutdruckwerte, ebenfalls als eine Bluthochdruck Prävention angesehen werden.

Ein Verdacht auf Prädiabetes liegt auch vor, wenn der Nüchtemblutzuckerwert über dem maximalen Normalwert von 110 mg Glucose pro dl Plasma liegt aber den für Diabetes relevanten Grenzwert von 125 mg Glucose pro dl Plasma nicht überschreitet. Ein anderes Indiz für Prädiabetes ist eine gestörte Glukose Toleranz, d.h. ein Blutzuckerwert von 140-200mg Glucose pro dl Plasma 2 Stunden nach nüchterner Einnahme von 75 g Glucose im Rahmen eines Glukose Toleranz Tests.

Auch ein Blutwert für Triglyzeride von mehr als 150 mg/dl lässt das Vorliegen von Prädiabetes vermuten. Dieser Verdacht wird durch niedrige Blutwerte für HDL-Cholesterin bestärkt. Bei Frauen sind Werte unter 40 mg pro dl Plasma, bei Männern Werte unter 50 mg pro dl Plasma als zu niedrig zu werten. Die Bestimmung von Triglyzeriden und HDL-Cholesterin im Blut gehört ebenfalls zu den Standardverfahren in der medizinischen Analytik und wird z.B. in: Thomas L (Hrsg.): Labor und Diagnose", TH-Books Verlagsgesellschaft mbH, Frankfurt/Main, 2000 beschrieben. Weiter bestärkt wird ein Verdacht auf Prädiabetes, wenn die Nüchternblutzuckerwerte gleichzeitig 110 mg Glucose pro dl Plasma überschreiten. Liegen die gemessenen Blutwerte im Bereich der Grenzwerte, so kann als zusätzliche Entscheidungshilfe das Verhältnis des Taillienumfangs zum Hüftumfang herangezogen werden. Überschreitet dieses Verhältnis bei Frauen den Wert 0,8 bzw. bei Männern den Wert 1 so ist eine Behandlung angezeigt.

Besonders angezeigt für die Behandlung von Diabetes bzw. vermuteter Prädiabetes sind die Angiotensin II Rezeptor Antagonisten Irbesartan und Telmisartan dann, wenn auch eine Hypertonie behandelt werden muss. Dies ist der Fall, wenn der systolische Blutdruck einen Wert von 140 mm Hg und der diastolische Blutdruck einen Wert von 90 mm Hg übersteigt. Leidet ein Patient bereits an manifester Diabetes wird heute empfohlen den systolischen Blutdruck auf einen Wert unter 130 mm Hg und den diastolische Blutdruck auf einen Wert unter 80 mm Hg zu senken. Um diese Werte zu erreichen kann von Fall zu Fall die Kombination des Angiotensin II Rezeptor Antagonisten mit einem Diuretikum oder einem Calcium Antagonisten angezeigt sein. Der Begriff "Diuretikum" umfasst Thiazide bzw. Thiazidanaloga wie Hydrochlorothiazide (HCTZ), Clopamide, Xipamide oder Chlorthalidone, Aldosteron-Antagonisten wie Spironolacton oder Eplerenone als auch andere Diuretika, die sich zur Behandlung von erhöhtem Blutdruck eignen wie Furosemide und Piretanide, und deren Kombinationen mit Amiloride und Triamteren.

Die vorliegende Erfindung bedeutet, dass für Personen, die wegen eines erhöhten Blutdrucks behandelt werden, die Angiotensin II Rezeptor Antagonisten Irbesartan und Telmisartan immer dann indiziert sind, wenn der Entwicklung von Diabetes vorgebeugt bzw. ein manifester Diabetes behandelt werden soll.

In nur 10 Prozent aller Fälle von erhöhtem Blutdruck (sekundäre Hypertonie) ist eine identifizierbare Ursache feststellbar, wie z.B. eine Nierenerkrankung. Durch Behandlung und Beseitigung der Ursache lässt sich die sekundäre Hypertonie in der Regel beheben. In fast 90 Prozent aller Fälle handelt es sich aber um eine primäre Hypertonie, deren genaue Ursache nicht bekannt und eine direkte Heilung somit nicht möglich ist. Die negativen Auswirkungen des erhöhten Blutdrucks können durch Veränderung der Lebensgewohnheiten und eine richtige Behandlung vermindert werden. Das Zusammenspiel verschiedener bzw. das gemeinsame Auftreten einzelner Risikofaktoren scheint den Bluthochdruck zu verursachen.
Vor allem das Zusammentreffen von Bluthochdruck mit Störungen des Fett- und Zuckerstoffwechsels wird vermehrt beobachtet. Diese Störungen bleiben zunächst oft unbemerkt, können aber anhand erhöhter Blutwerte von Triglyzeriden und Glucose sowie erniedrigten Blutwerten von HDL-Cholesterin erkannt werden. In etwas fortgeschrittenerem Stadium sind sie zusätzlich an einer langsam zunehmenden Fettleibigkeit zu erkennen. Erklären lassen sich diese Störungen durch zunehmende Insulin Resistenz. Je weniger wirksam das Insulin ist, desto mehr gerät der Fett- und Zuckerstoffwechsel durcheinander. Die Kombination all dieser Störungen erhöht letztlich die Wahrscheinlichkeit, an der Zuckerkrankheit Diabetes zu leiden und vorzeitig an einer Herz- oder Gefäßerkrankung zu sterben.

Schätzungen gehen davon aus, dass etwa ein Drittel der Erwachsenen in den Regionen der Erde, die mit Nahrung überversorgt sind, vom Zusammentreffen eines erhöhten Blutdrucks mit Störungen des Fett- und Zuckerstoffwechsels betroffen sind und dass diese Zahl noch zunehmen wird. Daraus resultiert ein Bedarf an Arzneimitteln, die in der Lage sind dazu beizutragen, das Fortschreiten der genannten Stoffwechselstörungen in einer möglichst frühen Phase zu verlangsamen oder zu stoppen und gleichzeitig die gesundheitsschädlichen Auswirkungen eines erhöhten Blutdrucks zu vermeiden.

Die vorliegende Erfindung offenbart auch ein Arzneimittel, das sowohl zur Behandlung von Hypertonie als auch zur Behandlung eines manifesten Typ 2 Diabetes bzw. erster Anzeichen der komplexen Stoffwechselstörung des Prädiabetes verwendet werden kann. Somit umfasst die vorliegende Erfindung auch die Diabetes Prävention in Patienten, die wegen erhöhtem Blutdruck behandelt werden. Wird daher einer der Angiotensin II Rezeptor Antagonisten Irbesartan und Telmisartan sofort dann zur Kontrolle des Blutdrucks verwendet, wenn eines der genannten Anzeichen auf Prädiabetes vorliegt, kann dadurch das Auftreten eines manifesten Typ 2 Diabetes verzögert oder vermieden werden.

Bei den im Rahmen der vorliegenden Erfindung geeigneten Angiotensin II Rezeptor Antagonisten Irbesartan und Telmisartan handelt es sich um Verbindungen, für die eine Bindung an die PPARgamma Ligandenbindungsdomäne durch *in vitro* Tests (vgl. Beispiel 1) ausgeschlossen werden kann, während sie zellulär, d.h. nach Zugabe zum Kulturmedium einer stabil transformierten PPARgamma-Reporterzelllinie, die Expression eines ebenfalls stabil transfizierten Luciferase Gens aktivieren (vgl. Beispiel 3).

Irbesartan und Telmisartan zeigen also
- keine *in vitro* Bindung an die Ligandenbindungsdomäne eines humanen PPARgamma Rezeptors, führen aber zur
- Induktion einer Luciferase Aktivität, wenn sie zum Kulturmedium einer stabil transformierten PPARgamma-Reporterzelllinie zugegeben werden, welche
   a) ein Fusionsprotein bestehend aus der Ligandenbindungsdomäne des humanen PPARgamma Transkriprionsfaktors sowie der Hefe GAL4 DNA Bindungsdomäne exprimiert und
   b) ein Luciferase Gen unter der Kontrolle einer fünffach wiederholten Hefe Gal4-Bindungsstelle enthält.
Die Herstellung einer solchen PPARgamma-Reporterzelllinie wird in Beispiel 2 beschrieben.

Eine *in vitro* Bindung an die Ligandenbindungsdomäne des humanen PPARgamma2 Rezeptors liegt dann nicht vor, wenn sie in einem AlphaScreen (Ullmann EF et al, Proc Natl Acad Sei USA (1994) 91:5426-5430) nicht nachgewiesen werden kann. Anstelle eines Alpha Screens kann auch ein SPA-Assay (Mukherjee R et al., J Steroid Biochem Mol Biol (2002) 81:217-225) oder eine NMR-Untersuchung (Johnson BA et al., J Mol Biol (2000) 298:187-194) durchgeführt werden. In der Regel kann mit keinerm dieser Verfahren eine Bindung an den Rezeptor nachgewiesen werden.

Angiotensin II Rezeptor Antagonisten im Rahmen der vorliegenden Erfindung sind Irbesartan und Telmisartan. Die besten Ergebnisse werden klar mit Telmisartan und dessen Salzen erzielt. Die hergestellten Darreichungsformen enthalten ein Äquivalent von 20-200 mg, vorzugsweise von 20, 40, 80, 120, 160 oder 200 mg der freien Säure des Wirkstoffs. Ist der Wirkstoff mit HCTZ oder Chlorthalidone kombiniert, so enthält die Darreichungsform 10-50 mg, vorzugsweise 50, 25 oder 12,5 mg des Diuretikums.

Die im Rahmen dieser Erfindung offenbarte vorteilhafte Wirkung der Angiotensin II Antagonisten Irbesartan und Telmisartan ist für Telmisartan besonders ausgeprägt. Erscheint die kombinierte Anwendung eines Angiotensin II Rezeptor Blockers mit ein oder mehreren anderen therapeutischen Wirkstoffen sinnvoll oder notwendig, stellt Telmisartan einen bevorzugten Angiotensin II Rezeptor Blocker dar, weil er in einem einzigen Wirkstoff eine Blutdruck-senkende mit einer metabolischen Wirkung kombiniert, z.B. einer antidiabetischen Wirkung die auch zur Prävention von Diabetes beiträgt.

Aus diesem Grund bedeuten vorformulierte Wirkstoffkombinationen von Telmisartan mit HMG-Co A Reduktase Inhibitoren wie Simvastatin oder Atorvastatin eine wegweisende Weiterentwicklung in der Therapie von kardiovaskulären, kardiopulmonalen, pulmonalen oder renalen Krankheiten, aber auch bei der Behandlung von Dyslipidämie, Osteoporose oder Alzheimer. Dies gilt auch für Wirkstoffkombinationen von Telmisartan mit Rosiglitazone oder Pioglitazone oder Repaglinide oder Metformin oder einem DPP4 Inhibitor in der Diabetes-Therapie. Auch bei der Behandlung von Bluthochdruck mit Inhibitoren des Renin-Angiotensin Systems (RAS) in Kombination mit einem Calcium Antagonisten wie Amlodipine oder Nifedipine oder einem Aldosterone Antagonisten wie Spironolacton oder Eplerenone muss Telmisartan als ein bevorzugter RAS Inhibitor angesehen werden. Die Kombination mit einem Aldosteron Antagonisten wie Eplerenone stellt auch für die Behandlung bzw. Prävention von Herzschwäche oder Herzinfarkt eine bedeutsame Weiterentwicklung dar.
Neben erhöhtem Blutdruck bedeuten auch Fettstoffwechselstörungen (Dyslipidämien) und Diabetes mellitus ein erhöhtes Schlaganfallrisiko, sodass Telmisartan auch in Verbindung mit Thrombocyten-Aggregationshemmem wie Clopidogrel oder Dipyridamol allenfalls zusätzlich kombiniert mit Acetylsalicylsäure (ASA) einen bevorzugten Kombinationspartner vor allem für die Schlaganfallprophylaxe darstellt. Dipyridamol kann zu diesem Zweck in einer Dosis von 50 bis 750 mg, vorzugsweise von 100 bis 500 mg und insbesondere von 200 bis 450 mg eingesetzt werden. ASA kann in einer Dosis von 10 bis 200 mg, vorzugsweise von 25 bis 100 mg und insbesondere von 30 bis 75 mg eingesetzt werden.

Entsprechende pharmazeutische Zusammensetzungen umfassen Telmisartan oder eines seiner Salze in Kombination mit
- Amlodipine oder Nifedipine,
- Eplerenone oder Spironolacton,
- Simvastatin oder Atorvastatin,
- Rosiglitazone oder Pioglitazone oder Repaglinide oder Metformin,
- Dipyridamol oder Clopidogrel, allenfalls in Kombination mit Acetylsalicylsäure,
- einem Sulfonylharnstoff,
- einem Aldosteron Antagonisten,
- einem HMG-Co A Reduktase Inhibitor,
- einem DPP4 Inhibitor oder
- einem Thrombocyten-Aggregationshemmer.
Diese vorformulierten Wirkstoffkombinationen werden in der Regel mit einem oder mehreren Formulierungshilfsstoffe wie Mannitol, Sorbitol, Xylit, Saccharose, Calciumcarbonat, Calciumphosphat, Lactose, Croscarmellose Natriumsalz (Cellulose carboxymethylether Natriumsalz, quervemetzt), Crospovidone, Natriumstärkeglykolat, Hydroxypropylcellulose (niedrigsubstituiert), Maisstärke, Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke, Magnesiumstearat, Natriumstearylfumarat, Talk, Hydroxypropylmethylcellulose, Carboxymethylcellulose, Celluloseacetatphthalat, Polyvinylacetat, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen eingearbeitet.

Tabletten erhält man z.B. durch Mischen des oder der Wirkstoffe mit ein oder mehreren Hilfsstoffen und anschliessende Verpressung. Die Tabletten können auch aus mehreren Schichten bestehen. Beispiele für Hilfsstoffe sind
- inerte Verdünnungsmitteln wie Mannitol, Sorbitol, Xylit, Saccharose, Calciumcarbonat, Calciumphosphat und Lactose;
- Sprengmitteln wie Croscarmellose Natriumsalz (Cellulose carboxymethylether Natriumsalz, quervernetzt), Crospovidone, Natriumstärkeglykolat, Hydroxypropylcellulose (niedrigsubstituiert) und Maisstärke;
- Bindemitteln wie Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke;
- Schmiermitteln wie Magnesiumstearat, Natriumstearylfumarat und Talk;
- Mitteln zur Erzielung des Depoteffektes wie Hydroxypropylmethylcellulose, Carboxymethylcellulose, Celluloseacetatphthalat und Polyvinylacetat; und
- Pharmazeutisch zulässige Farbstoffe wie farbige Eisenoxide
Blockade von Aldosteron reduziert die Mortalität und Morbidität bei Patienten mit ausgeprägter Herzschwäche. Erleiden Patienten, die wegen symptomatischer Herzschwäche und Linksherzinsuffizienz mit einem ACE Hemmern, Angiotensin Rezeptor Antagonisten, Diuretikum oder Beta-Blocker behandelt werden, einen Myocard Infarkt, so konnte gezeigt werden, dass die zusätzliche Behandlung mit dem selektiven Aldosteron Blocker Eplerenone ihre Überlebenschancen verbessert und zu einer Verminderung späterer Spitalseinweisungen führt (NEJM 348:1309-1321, 2003). In der untersuchten Patientengruppe wurden auch Diabetespatienten miterfasst, bei denen zwar eine Linksherzinsuffizienz aber keine symptomatische Herzschwäche vorlag, da für diese Patientengruppe das Risiko eines kardiovaskulären Ereignisses vergleichbar gross ist wie bei Patienten mit Linkshersinsuffizienz und symptomatischer Herzschwäche. Berücksichtigt man dies, so bedeutet die hier offenbarte Entdeckung der antidiabetischen Wirkung des Angiotensin II Rezeptor Blockers Telmisartan, dass seine Kombination mit Eplerenone bei der Nachbehandlung eines Myocard Infarktes vor allem bei Patienten angezeigt ist, die bereits an Diabetes und allenfalls diabetischer Proteinurie leiden oder bei denen Verdacht auf Prädiabetes besteht. Pharmazeutische Darreichungsformen, in denen die beiden Wirkstoffe Telmisartan und Eplerenone kombiniert vorliegen, können beispielsweise
ein Äquivalent von 40-320 mg, vorzugsweise 80 oder 160 mg Telmisartan und ein Äquivalent von 20-200 mg, vorzugsweise 25 oder 50 mg Eplerenone enthalten. Eine bevorzugte Darreichungsform sind Zweischichttabletten. Zur Behandlung solcher Patienten mit gleichzeitigen Anzeichen einer Dyslipidämie (z.B. Hypertriglyceridämie oder Hypercholesterolämie) ist die zusätzliche Kombination mit einem Lipidsenker wie Simvastatin oder Atorvastatin in der üblichen Dosierung von 2.5-40 mg, vorzugsweise 5, 10, 15, 20, 25, 30, 35 oder 40 mg häufig sinnvoll. Eine entsprechende Darreichungsform von Telmisartan, Eplerenone und Atorvastatin oder Simvastatin kann z.B. als Dreischichttablette hergestellt werden. Der Wirkstoff Eplerenone wird vor allem in mikronisierter Form mit einer D₉₀ Teilchengrösse von 25-400 Micron verwendet (vgl. WO 00/33847).

Metformin ist ein bewährtes Antidiabetikum, das seinen Haupteffekt dadurch erzielt, dass es die überhöhte Glucoseproduktion in der Leber eines Diabetikers senkt. In der Therapieüberwachung des Diabetes mellitus hat der HbA1c-Wert, das Produkt einer nicht-enzymatischen Glykierung der Hämoglobin B-Kette, eine überragende Bedeutung. Da seine Bildung im wesentlichen von der Blutzuckerhöhe und der Lebenszeit der Erythrozyten abhängt, reflektiert der HbA1 c im Sinne eines "Blutzuckergedächtnisses" den mittleren Blutzuckerspiegel der vergangenen 4-6 Wochen. Diabetische Patienten, deren HbA1c-Wert durch intensivierte Diabetestherapie dauerhaft gut eingestellt ist (d.h. < 6.5 % des gesamten Hämoglobins der Probe), sind deutlich besser vor diabetischer Mikroangiopathie geschützt. Metformin alleine erreicht beim Diabetiker eine durchschnittliche Verbesserung des HbA1 c Wertes in der Größenordnung von 1.0 - 1.5 %. Diese Reduktion des HbA1 C Wertes ist nicht bei allen Diabetikern ausreichend um in den gewünschten Zielbereich von < 6.5 % und bevorzugt < 6 % HbA1c zu kommen. Deshalb werden zusätzliche therapeutische Maßnahmen erforderlich, um die Wirkung von Metformin zu verstärken.

Im Rahmen der vorliegenden Erfindung wurde überraschend gefunden, dass Telmisartan als Aktivator von PPAR-gamma wirkt. Somit besitzt Telmisartan das Potential, die Insulinsensitivität von Fett-, Muskel- und Lebergewebe zu steigern, und auf diesem Weg eine Blutzuckersenkung zu erreichen. Dies macht Telmisartan zu einem besonders geeigneten Kombinationspartner für Antidiabetika wie Metformin oder Repaglinide (fördert die Freisetzung von Insulin aus den B-Zellen der Bauchspeicheldrüse), weil seine Wirkung auf einem anderen Wirkprinzip beruht und somit die Wirkweise dieser Wirkstoffe günstig verstärkt. Darreichungsformen einer Kombination von Repaglinide und Telmisartan enthalten beispielsweise ein Äquivalent von 0.25 bis 5 mg, vorzugsweise 0.25 bis 2 mg, und besonders bevorzugt 0.5 oder 1 oder 2 mg Repaglinide und ein Äquivalent von 40-320 mg, vorzugsweise 80 oder 160 mg Telmisartan.

Eine Kombination von Telmisartan und Metformin ist besonders geeignet beim adipösen Typ 2 Diabetiker, da einerseits Metformin im Unterschied zu anderen oralen Antidiabetika nicht zu einer Steigerung des Körpergewichts führt, und zum anderen Telmisartan die Insulinresistenz als wichtiges Merkmal und Ursache der erhöhten Blutzuckerspiegel bei diesen Typ 2 Diabetikern reduziert. Bei den meisten adipösen Typ 2 Diabetikern wie auch Prädiabetikern wird eine Erhöhung des Blutdruckes festgestellt, der auch zu den Kriterien des Metabolischen Syndroms gehört. Für diese Patientengruppe bedeutet Telmisartan ein bevorzugtes Antihypertensivum dessen zusätzliche Eigenschaften als Insulin Sensitizer mit einem bewährten Antidiabetikum wie Metformin günstig zusammenwirken, um gleichzeitig und gleichermaßen verschiedene Aspekte der Erkrankungen Diabetes Typ 2, Prädiabetes Typ2 oder Metabolisches Syndrom oder Insulinresistenz zu behandeln. Durch die ergänzende Gabe von Telmisartan kann eine zusätzliche Verbesserung des HbA1 c Wertes in der Größenordnung von 0.25 - 2 %, vorzugsweise 0.25 - 1 % und besonders bevorzugt 0.25 - 0.5 % erreicht werden. Auch eine Verbesserung des HbA1c Wertes von weniger als 0.25 % des gesamten Hämoglobins bedeutet einen sinnvollen Beitrag zur Behandlung eines Typ 2 Diabetikers, ist derzeit aber nicht verlässlich messbar. Wie die UKPDS (United Kingdom Prospective Diabetes Study) Studie gezeigt hat, ist eine Reduktion des überhöhten Blutdruckes ebenso wirksam wie eine blutzuckersenkende Therapie, um bei Typ 2 Diabetikern Spätkomplikationen wie Nephropathie, Neuropathie, Retinopathie wie auch alle makrovaskulären Komplikationen zu reduzieren. Damit bedeutet die vorgeschlagene Kombination von Telmisartan und Metformin einen wesentlichen Beitrag zur Reduktion oder sogar Verhinderung der schwerwiegenden Folgeerscheinungen des Diabetes.

Darreichungsformen einer Kombination von Metformin und Telmisartan enthalten beispielsweise ein Äquivalent von 450-900 mg, vorzugsweise 500 oder 850 mg Metformin und ein Äquivalent von 40-320 mg, vorzugsweise 80 oder 160 mg Telmisartan. Metforminhydrochlorid ist gut löslich und lässt sich unproblematisch mit Hilfsstoffen wie Bindemitteln und Schmiermitteln formulieren. Eine bevorzugte Darreichungsform sind Zweischichttabletten. Die Kombination der bevorzugten Wirkstoff- und Hilfsstoffmengen resultiert in folgenden Massen:

**Tabelle 1: Tabletten mit 80 mg Telmisartan**

| | **Telmisartan/metformin** | |
|---|---|---|
| | 80/500 mg | 80/850 mg |
| Metforminhydrochlorid (entsprechend 500 mg bzw. 850 mg Metformin) | 643 mg | 1094 mg |
| Hilfsstoffe (Bindemittel und Schmiermittel) mindestens | 27 mg | 46 mg |
| Telmisartan SD-Granulat (entsprechend 80 mg Telmisartan) | 135 mg | 135 mg |
| Hilfsstoffe aus Tablettenmatrix Telmisartan (Sorbitol und Schmiermittel) | 345 mg | 345 mg |
| **Summe Tablette** (mindestens) | **1150 mg** | **1620 mg** |

**Tabelle 2: Tabletten mit 160 mg Telmisartan**

| | **Telmisartan/metformin** | |
|---|---|---|
| | 160/500 mg | 160/850 mg |
| Metforminhydrochlorid (entsprechend 500 mg bzw. 850 mg Metformin) | 643 mg | 1094 mg |
| Hilfsstoffe (Bindemittel und Schmiermittel) mindestens | 27 mg | 46 mg |
| Telmisartan SD-Granulat (entsprechend 160 mg Telmisartan) | 270 mg | 270 mg |
| Hilfsstoffe aus Tablettenmatrix Telmisartan (Sorbitol und Schmiermittel) | 690 mg | 690 mg |
| **Summe Tablette** (mindestens) | **1630 mg** | **2100 mg** |

Darreichungsformen einer Kombination von Repaglinide und Telmisartan enthalten beispielsweise ein Äquivalent von 0.25 bis 5 mg, vorzugsweise 0.25 bis 2 mg, und besonders bevorzugt 0.5 oder 1 oder 2 mg Repaglinide und ein Äquivalent von 40-320 mg, vorzugsweise 80 oder 160 mg Telmisartan.

### Beispiele:

### Beispiel 1: Telmisartan, Losartan* und Irbesartan binden in vitro nicht an die PPARgamma Ligandenbindungsdomäne (*Referenzbeispiel)

Protein enthaltend die humane PPARgamma-Ligandenbindungsdomäne (LBD) wird als GST-Fusionsprotein in E.coli hergestellt und affinitätschromatographisch gereinigt.
Hierzu wird ein DNA Abschnitt, der für die Aminosäuren 205-505 des humanen PPARgamma2-Transkriptionsfaktors kodiert (vgl. Genbank-Eintrag U79012) über die zusätzlich eingefügten Restriktionsschnittstellen BamH I und Xho I in den Expressionsvektor pGEX-4T-1 (Amersham) subkloniert und die Sequenz des Abschnitts kontrolliert. Die Expression des Fusionsproteins erfolgt im für pGEX Vektoren empfohlenen E.coli-Stamm BL21(DE3) nach Induktion mit 0.2mM IPTG für 4 Stunden bei 25°C. Die Bakterien werden im Anschluss an die Induktion pelletiert und portionsweise in PBS, pH7.4 weggefroren. Nach Aufschluß in einer French Press, wirde das gelöste GST-PPARgamma-LBD-Fusionsprotein mit Hilfe einer GSTrap-Säule (Pharmacia) gereinigt. Die Elution erfolgte durch Zugabe von 20mM reduziertem Glutathion.
Die GST-PPARgamma-LBD-Proteinfraktionen werden mit Hilfe einer HiTrap Desalting-Säule (Pharmacia) entsalzt und die Proteinkonzentration mit einem Standardassay bestimmt.

Protein enthaltend die humane RXRalpha-Ligandenbindungsdomäne (LBD) wird als His tag-Fusionsprotein in E.coli hergestellt und affinitätschromatographisch gereinigt. Hierzu wird ein DNA-Abschnitt, der für die Aminosäuren 220-461 des humanen RXRalpha-Transkriptionsfaktors kodiert (vgl. Genbank-Eintrag NM_002957, nt 729-1457) über die zusätzlich eingefügten Restriktionsschnittstellen BamH I und Not I in den Expressionsvektor pET28c (Novagen) subkloniert und die Sequenz des Abschnitts kontrolliert. Die Expression des Fusionsproteins erfolgt im für pET-Vektoren empfohlenen E.coli-Stamm BL21(DE3) nach Induktion mit 0.2mM IPTG für 4 Stunden bei 25°C. Die Bakterien werden im Anschluss an die Expression pelletiert und portionsweise in PBS, pH7.4 weggefroren. Nach Aufschluß in einer French Press, wird das gelöste His-RXRalpha-LBD-Fusionsprotein mit Hilfe einer HiTrap Chelating-Säule (Pharmacia) gereinigt. Die Elution erfolgte durch eine 500mM Imidazol-Stufe. Die His-RXRalpha-LBD-Proteinfraktionen werden mit Hilfe einer HiTrap Desalting-Säule (Pharmacia) entsalzt und die Proteinkonzentration mit einem Standardassay bestimmt.

### a) AlphaScreen

Alpha Screen-Assays wurden erstmals in Ullmann EF et al, Proc Natl Acad Sci USA (1994) 91:5426-5430 beschrieben. Die im Rahmen dieses Beispiel durchgeführten Messungen erfolgten wie bei Glickman JF et al., J Biomol Screen (2002) 7:3-10 beschrieben. Der Assay-Puffer besteht aus 25mM Hepes pH7.4, 100mM NaCl, 1 mM DTT, 0.1 % Tween-20, 0.1 % BSA. 3nM GST-PPARgamma-LBD-Fusionsprotein, 15nM biotinyliertes LXXLL-Peptid des Cofaktros CBP (entsprechend dem auf Seite 218 von Mukherjee R et al., J Steroid Biochem Mol Biol (2002) 81:217-225 offenbarten Peptid mit einem zusätzlichen N-terminalen Cystein) und jeweils 10µg/ml anti-GST-Akzeptorbeads bzw. Streptavidin-Donorbeads (Applied Biosystems) werden in einem Gesamtvolumen von 12.5µl in Gegenwart von unterschiedlichen Konzentrationen einer Testsubstanz (in DMSO) für 4 Stunden bei Raumtemperatur inkubiert. Die finale DMSO-Konzentration im Assay beträgt 1 % (v/v). Als Hintergrundkontrolle (NSB) dient eine 1 %ige DMSO-Lösung. Die Messung erfolgt am Packard Fusion-Messgerät.

| | **Telmisartan** | | **Rosiglitazone** | |
|---|---|---|---|---|
| Konz. / M | MW | SD | MW | SD |
| NSB | 619 | 21 | 573 | 17 |
| 1,00E-08 | | | 820 | 18 |
| 3,00E-08 | 642 | 41 | 1720 | 48 |
| 1,00E-07 | 606 | 10 | 8704 | 59 |
| 3,00E-07 | 644 | 56 | 27176 | 1232 |
| 1,00E-06 | 677 | 14 | 43233 | 1083 |
| 3,00E-06 | 720 | 35 | 52691 | 3771 |
| 1,00E-05 | 847 | 82 | 56366 | 4303 |
| 5,00E-05 | 1111 | 135 | | |

Im Gegensatz zu Rosiglitazone, einem literaturbekannten PPARgamma-Agonisten mit Bindung in der LBD, findet durch zunehmende Konzentrationen von Telmisartan, Losartan und Irbesartan (Konzentrationen bis 50µM) keine direkte Aktivierung der PPARgamma-LBD und damit verbunden keine signifikante Rekrutierung des LXXLL-Peptids statt.

### b) SPA-Assay

Eine Beschreibung des SPA-Assayformats findet sich in Mukheriee R et al., J Steroid Biochem Mol Biol (2002) 81:217-225. Der Assaypuffer besteht aus 20mM Tris pH7.5, 25mM KCI, 10mM DTT und 0.2% Triton X-100. 30nM GST-PPARgamma-LBD-Fusionsprotein, 30nM His-RXRalpha-LBD, anti-GST-Antikörper (1:600, Amersham Pharmacia), 0.25mg Protein A SPA PVT Antibody-binding beads (Amersham Pharmacia), 30nM ³H-markiertes Rosiglitazone werden in einem Gesamtvolumen von 100µl mit Testsubstanzverdünnungen für 5 Stunden bei Raumtemperatur inkubiert.
Als Hintergrundkontrolle (NSB) wird 10µM unmarkiertes Rosiglitazone anstelle des radioaktiven Rosiglitazones zugesetzt, , als Maximalwert (Bmax) wird anstelle einer Testsubstanz das verwendete Lösungsmittel, z.B. DMSO, zugegeben.

Im Anschluss an die Inkubation werden die Testansätze für 5 Minuten bei 2000 Upm in einer Hettich Universal 30Rf-Zentrifuge abzentrifugiert und am Packard TopCount NXT gemessen.

| | **Telmisartan** | | **Irbesartan** | | **Losartan** | |
|---|---|---|---|---|---|---|
| Konz / M | MW | SD | MW | SD | MW | SD |
| NSB | 217 | 9 | 217 | 9 | 217 | 9 |
| Bmax | 911 | 15 | 911 | 15 | 911 | 15 |
| 1,00E-07 | 837 | 49 | 913 | 54 | 915 | 43 |
| 3,00E-07 | 802 | 28 | 810 | 49 | 835 | 11 |
| 1,00E-06 | 818 | 27 | 815 | 51 | 901 | 10 |
| 3,00E-06 | 818 | 20 | 779 | 26 | 814 | 53 |
| 1,00E-05 | 703 | 30 | 723 | 37 | 787 | 46 |
| 3,00E-05 | 691 | 222 | 648 | 40 | 784 | 96 |
| 1,00E-04 | 545 | 18 | 510 | 81 | 611 | 17 |

Im Gegensatz zu direkten PPARgamma-Agonisten, die an die PPARgamma-LBD binden, findet auch bei sehr hohen Überschüssen von Telmisartan, Losartan oder Irbesartan keine konzentrationsabhängige Verdrängung des radioaktiven Rosiglitazones aus der Bindungstasche statt.

### c) NMR-Untersuchungen

Im Gegensatz zu einem direkten PPARgamma-Liganden, z.B. Rosiglitazone, findet bei der Messung des ¹⁵N TROSY-Spektrums der PPARgamma-LBD in Gegenwart der Testsubstanzen Telmisartan keine Wechselwirkung der Testsubstanz mit Aminosäuren der Bindungstasche statt. Die Aminosäuren der Bindungstasche weisen in Gegenwart der Testsubstanzen die gleiche Positionierung auf, wie in Abwesenheit eines Liganden.

### Beispiel 2: Herstellung einer stabil transformierten PPARgamma-Reporterzelllinie

Ein DNA Abschnitt, der für die Aminosäuren 205-505 des humanen PPARgamma2-Transkriptionsfaktors kodiert (entsprechend den Nukleotiden 703-1605 der Genbank-Sequenz U79012) wird über zusätzlich eingefügte Restriktionsschnittstellen BamH I und Hind III in die Multiple Cloning Site des Vektors pFA-CMV (Stratagene) eingebaut und die Sequenz verifiziert. Das resultierende Plasmid pFA-CMV/hPPARgamma2-LBD kodiert N-terminal der PPARgamma-LBD im gleichen Leseraster für eine Gal4 DNA-Bindungsdomäne. Zusätzlich kodiert das Plasmid für eine Neomycin-Resistenz.

Die Zelllinie CHO-K1 (ATCC CCL-61 ) wird mit den Plasmiden pFA-CMV/hPPARgamma2-LBD und pFR-Luc (Stratagene) cotransfiziert. pFR-Luc kodiert für das Luciferase-Gen unter der Kontrolle einer fünffach wiederholten Hefe Gal4-Bindungsstelle. Die Transfektion wird mit Lipofectamin2000 gemäß Herstellerangaben durchgeführt.
Nach der Transfektion werden die Zellen in Medium (Ham's F12 mit 10% fötalem Kälberserum) in Gegenwart von 0.5 mg/ml G-418 kultiviert. Nach sechstägiger Kultivierung werden die Zellen passagiert und für weitere 10 Tage in Kultur gehalten. Die resultierenden Neomycin-resistenten Kolonien werden unter dem Mikroskop gepickt und in 96-Well-Platten vereinzelt und kultiviert. Man erhält verschiedene transformierte Zelllinien mit den enthaltenen Plasmiden (z.B. Klon Nr. 10, 11, 13 etc), die weiter im Kulturmedium gehalten werden.

Die Zelllinien werden hinsichtlich der Induzierbarkeit des Luziferase Gens durch einen PPARgamma-Agonisten, z.B. Rosiglitazone, untersucht und reagieren mit einem gesteigerten Luciferasesignal auf Stimulation durch den PPARgamma-Agonisten.

### Beispiel 3: Telmisartan, Losartan* und Irbesartan aktivieren PPARgamma zellulär (*Referenzbeispiel)

Die vom transformierten Klon 11 des Beispiels 2 abgeleitete CHO-K1 Zelllinie wird in 96-well-Flachbodenplatten in einer Dichte von 3x10⁴ Zellen/200µl/well ausgesät und über Nacht in Ham's F-12-Medium mit 10% fötalem Kälberserum und 0.5mg/ml G-418 kultiviert. Nach 24 Stunden wird auf Medium ohne Zusatz von G-418 gewechselt.
Die Testsubstanzen werden mit einem geeigneten Lösungsmittel, z.B. DMSO, auf das 100fache der gewünschten Konzentration gebracht und durch das vorgelegte Medium der Zellkulturplatte 1:100 verdünnt. Als Hintergrundkontrolle dient das verwendete Lösungsmittel, z.B. DMSO, in gleicher Konzentration.
24 Stunden nach Substanzgabe werden die Überstände verworfen und die Zellen je zweimal mit 150µl Waschpuffer (25mM Tricine, 16.3mM MgSO₄, pH7.8) gewaschen. Nach den Waschschritten werden je Testansatz 50µl Waschpuffer mit 150µl Luziferase-Assaypuffer (25mM Tricine, 0.5mM EDTA, 0.54mM NaTPP, 16.3mM MgSO₄, 1.2mM ATP, 0.05mM Luciferin, 56.8mM 2-Mercaptoethanol, 0.1% Trition X-100, pH7.8) zugesetzt. Die Messung der Lumineszenz erfolgt nach fünfminütiger Wartezeit am Packard TopCount NXT. Die Luziferase-Aktivität wird durch die Integration der relativen Luziferaseeinheiten (RLU) der ersten zehn Sekunden nach Start der Messung erhalten.

| | **Telmisartan** | | **Irbesartan** | | **Losartan** | | **Rosiglitazone** | |
|---|---|---|---|---|---|---|---|---|
| Konz / M | MW | SD | MW | SD | MW | SD | MW | SD |
| NSB | 466 | 188 | 466 | 188 | 466 | 188 | 741 | 141 |
| 1,00E-08 | | | | | | | 2761 | 178 |
| 3,00E-08 | | | | | | | 8256 | 708 |
| 1,00E-07 | | | | | | | 35265 | 2947 |
| 3,00E-07 | 760 | 255 | 491 | 70 | 874 | 475 | 86859 | 6139 |
| 1,00E-06 | 2859 | 455 | 657 | 65 | 589 | 70 | 106252 | 30018 |
| 3,00E-06 | 24498 | 2290 | 1028 | 342 | 672 | 88 | 143232 | 14064 |
| 1,00E-05 | 61397 | 7853 | 3292 | 556 | 709 | 163 | 150989 | 24245 |
| 3,00E-05 | 58790 | 2055 | 22133 | 4202 | 3271 | 585 | | |
| 1,00E-04 | | | 29600 | 6936 | 11322 | 1668 | | |

Durch den Angiotensin II Rezeptor Antagonisten Telmisartan erfolgt eine besonders starke Aktivierung des PPARgamma-Pathways in der PPARgamma-Reporterzelllinie. Eine Aktivierung durch andere Angiotensin II Rezeptor Antagonisten wie Losartan und Irbesartan findet erst bei höheren Testkonzentrationen und in einem geringeren Ausmaß statt.

### Beispiel 4: Experimente mit 3T3-L1 Adipocyten und PC12W Zellen

3T3-L1 Maus-Präadipocyten werden in DMEM (Dulbecco's modified eagle medium) mit 10% fötalem Kälberserum (FBS) kultiviert. PC12W Zellen werden in DMEMmit 5% FBS and 10% Pferdeserum kultiviert. In beiden Fällen enthalten die Medien 1 % Penicillin / Streptomycin.
Die Differenzierung von Adipocyten wird 2-3 Tage nach Zellkonfluenz durch die Zugabe einer Differenzierungslösung induziert. Diese enthält
1µmol/L Dexamethasone,
0,5mmol/L 3-isobutyl-1-methylxanthine,
1,67µmol/L insulin, und
10% FBS.
Zum Vergleich wird die Differenzierung auch mit einer Differnzierungslösung induziert, die zusätzlich Telmisartan enthält. Nach 48 Stunden (Tag 2) wird das Medium durch DMEM enthaltend 10% FBS und 1,67 µmol/L insulin bzw. 10% FBS und 1,67 µmol/L insulin und Telmisartan ersetzt. Danach werden die Zellen weitere 48 Stunden stimuliert und bevor sie schliesslich analysiert werden (Tag 4).

### Lipidakkumulation in 3T3-L1 Adipocyten

Zellen werden mit PBS gewaschen und mit einer 3,7%-igen Formaldehyd Lösung für 2 Minuten fixiert. Nach der Fixierung werden die Zellen 1 Stunde lang bei Raumtermperatur mit einer 3:2 mit Wasser verdünnten 0,5%-igen Vorratslösung von Oil Red-O in Isopropanol eingefärbt. Nach dem Waschen werden die Zellen unter dem Lichtmikroskop untersucht.
10µmol/L Telmisartan bewirken eine gesteigerte Lipidakkumulation, die durch eine verstärkte Oil Red-O Färbung sichtbar wird. Die Differenzierung von 3T3-L1 Adipocyten wird also durch Telmisartan begünstigt.

### Stimmulation der aP2 Expression in 3T3-L1 Zellen

RNA Isolierung, Reverse Transkription und Quanitifizierung der Genexpression erfolgt mit einem ABI 7000 Sequenz Detetionssystem für Echtzeit PCR (beschrieben in Janke et al, Diabetes 51:1699-707, 2002). Als endogene Kontrolle für die Echtzeit PCR dienen die Haushaltsgene 18S rRNA und Hypoxanthin Guanin Phosphoribosyl Transferase (hprt).
Die beobachtete Induktion ist abhängig von der eingesetzten Konzentration Telmisartan. 10µmol/L Telmisartan stimmulieren die Expression des adipogenen Marker Gens Adipose Protein 2 (aP2) in 3T3-L1 Zellen um das 3,1±0,3-fache (p<0,01). Im Vergleich dazu stimuliert eine Konzentration von 10µmol/L des PPARgamma Liganden Pioglitazone die aP2 Expression um das 4,5±1-fache (p<0,01).

### Transkriptions-Reporter Assays

Um zu untersuchen, ob die Induktion der Adipogenese durch Telmisartan das Ergebnis einer Stimulation der PPARgamma Aktivität ist, werden Transfektionsexperimente mit PPRE (PPAR Response Element)-Reporter Konstrukten durchgeführt. Die transiente Transfektion und die verwendeten Luciferase Assays werden beschrieben in Kintscher et al, Circ Res. 91 :e35-44, 2002. 3T3-L1 Adipocyten (Tag 4) bzw. PC12W Zellen werden mit Lipofectamine 2000 (Invitrogen) in Gegenwart von 1µg (für 3T3-L1 Zellen) bzw. 50ng (für PC12W Zellen) eines Reporter Konstruktes, PPARgamma2 und RXRalpha Expressionsvektoren sowie 10ng eines Renilla Luciferase Reporter Kontrollvektors transfiziert. Beim Reporter Konstrukt handeltes sich um eine Fusion von 3xAcyl-CoA oxidase PPAR Response Element (PPRE) mit Tk-Luciferase. Die verwendeten PPARgamma2 und RXRalpha Expressionsvektoren entsprechen den bei Elbrecht et al, Biochem Biophys Res Com 224: 431-437, 1996 und Joseph et al, J Biol Chem 277(13): 11019-11025, 2002 beschriebenen Vektoren. Beim Luciferase Reporter Kontrollvektor handelt es sich um das Plasmid pRL-CMV (Promega). Nach 4 Stunden wird das Transfektionsmedium durch DMEM mit 10% FBS ersetzt, das zusätzlich Telmisartan, Pioglitazone oder den Träger DMSO enthält. Luciferase Aktivität wird nach 24 Stunden gemessen.

Behandlung von 3T3-L1 Adipocyten mit 10µmol/L Telmisartan führen zu einer Induktion der transkriptionellen Aktivität von PPARgamma um das 3,4±0,9-fache (p<0,05) verglichen mit einer Induktion um das 5,2±1,1-fache durch 10µmol/L Pioglitazone.

PC12W Zellen sind AT₁-Rezeptor-defizient. PPARgamma 2 und sein heterodimerer Partner RXRalpha werden in PC12W Zellen überexprimiert und die PPARgammaabhängige Transkription in Gegenwart bzw. Abwesenheit von 10µmol/L Telmisartan bzw. Pioglitazone gemessen. Da PC12W Zellen PPARgamma nicht exprimieren, wird in Abwsesenheit von exogenem PPARgamma2 / RXRalpha keine Regulation der PPARgamma Aktivität gemessen. Nach Überexpression des PPARgamma 2 /RXRalpha Heterodimers induziert Telmisartan aber auch in den AT₁-Rezeptor-defizienten PC12W Zellen die PPARgamma Aktivität um das 1,9±0,4-fache (p<0,05). Im Vergleich dazu induziert Pioglitazone die PPARgamma Aktivität um das 4,2±1,4-fache (p<0,01). Dies beweist, dass die Aktivierung der PPARgamma Aktivität durch Telmisartan unabhängig von der Blockierung des AT₁-Rezeptors erfolgt.

Die Daten bedeuten auch, dass Telmisartan Konzentrationen, die zur Stimulierung der PPARgamma Aktivität notwendig sind, im Blutplasma von Patienten erreicht werden können, die mit Telmisartan gegen Bluthochdruck behandelt werden. Die bedeutet, dass eine Bluthochdruck Behandlung mit Telmisartan zusätzlich die Insulin Sensitivität zu verbessern vermag, was sich positiv auf den Blutzuckerspiegel auswirkt.

### Beispiel 5: Formulierungsbeispiele

**Tablette 1**

| Durch direkte Verpressung des Telmisartan Natriumsalzes mit Hilfsstoffen und Magnesiumstearat werden Tabletten folgender Zusammensetzung erhalten: | |
|---|---|
| Bestandteile: | mg |
| Telmisartan-Natriumsalz | 41,708 |
| Mannitol | 149,542 |
| Mikrokristalline Cellulose | 50,000 |
| Croscarmellose Natriumsalz | 5,000 |
| Magnesiumstearat | 3,750 |
| Gesamt | 250,000 |

**Tablette 2**

| Durch direkte Verpressung des Telmisartan Natriumsalzes mit Hilfsstoffen und Magnesiumstearat werden Tabletten folgender Zusammensetzung erhalten: | |
|---|---|
| Bestandteile: | mg |
| Telmisartan-Natriumsalz | 83,417 |
| Sorbitol | 384,083 |
| Polyvidon K25 | 25,000 |
| Magnesiumstearat | 7,500 |
| Gesamt | 500,000 |

### Tablette 3

Hydrochlorothiazid, Telmisartan Natriumsalz, Sorbitol und rotes Eisenoxid werden in einem Freifallmischer ("Free Fall Blender") gemischt, durch ein 0,8 mm Sieb gesiebt und, nach Zugabe von Magnesium Stearat, in einem Freifallmischer zu einer pulverförmigen Mischung verarbeitet.

Diese Zusammensetzung von Wirkstoffen und Hilfsstoffen wird dann mit einer geeigneten Tablettenpresse (z.B. Korsch EK0 oder Fette P1200) zu Tabletten verpresst. Es werden Tabletten mit folgender Zusammensetzung hergestellt, wobei die pro Tablette enthaltene Menge Telmisartan Natriumsalz einer Menge von 80 mg der freien Säure des Telmisartans entspricht.

| **Inhaltstoff** | **mg/Tablette** | **%** |
|---|---|---|
| Telmisartan Natriumsalz | 83,417 | 13,903 |
| Hydrochlorothiazid | 12,500 | 2,083 |
| Sorbitol | 494,483 | 82,414 |
| Rotes Eisenoxid | 0,600 | 0,100 |
| Magnesiumstearat | 9,000 | 1,500 |
| **Total** | **600,000** | **100,000** |

Das Telmisartan Natriumsalze der Tabletten der drei Chargen löst sich nach 30 Minuten Rühren (75 rpm) in 900 ml 0,1 M Phosphatpuffer pH 7,5 zu 92 ± 1,5 %, 96 ± 1,8 % bzw. 100 ± 1,0 %. Das Hydrochlorothiazid löste sich nach 30 Minuten in 900 ml 0,1 M HCl (100 rpm) zu 69 ± 6,3 %, 72 ± 2,1 % bzw 78 ± 1,8 %.

## Patentansprüche

1. Pharmazeutische Zusammensetzung, umfassend einen Angiotensin II Rezeptor Antagonisten ausgewählt aus Irbesartan und Telmisartan oder ein Salz davon zur Verwendung in einem Verfahren zur Behandlung von Personen bei denen Diabetes Mellitus Typ 2 diagnostiziert wurde oder Verdacht auf Prädiabetes besteht oder zur Prävention von Diabetes.

2. Pharmazeutische Zusammensetzung zur Verwendung gemäß Anspruch 1, **dadurch gekennzeichnet dass** bei den zu behandelnden Personen der Nüchternblutzuckerwert 125 mg Glucose pro dl Plasma übersteigt.

3. Pharmazeutische Zusammensetzung zur Verwendung gemäß Anspruch 1, **dadurch gekennzeichnet dass** bei den zu behandelnden Personen der Nüchternblutzuckerwert 110-125 mg Glucose pro dl Plasma beträgt.

4. Pharmazeutische Zusammensetzung zur Verwendung gemäß Anspruch 1, **dadurch gekennzeichnet dass** bei den zu behandelnden Personen 2 Stunden nach nüchterner Einnahme von 75 g Glucose ein Blutzuckerwert über 200mg Glucose pro dl Plasma gemessen wird.

5. Pharmazeutische Zusammensetzung zur Verwendung gemäß Anspruch 1, **dadurch gekennzeichnet dass** bei den zu behandelnden Personen 2 Stunden nach nüchterner Einnahme von 75 g Glucose ein Blutzuckerwert von 140-200mg Glucose pro dl Plasma gemessen wird.

6. Pharmazeutische Zusammensetzung zur Verwendung gemäß Anspruch 1, **dadurch gekennzeichnet dass** bei den zu behandelnden Personen der Blutwert für Triglyceride 150 mg/dl überschreitet.

7. Pharmazeutische Zusammensetzung zur Verwendung gemäß Anspruch 6, **dadurch gekennzeichnet dass** bei den zu behandelnden Personen der Blutwert für HDL bei Frauen 40 mg pro dl Plasma, bei Männern 50 mg pro dl Plasma unterschreitet.

8. Pharmazeutische Zusammensetzung zur Verwendung gemäß den Ansprüchen 6 und 7, **dadurch gekennzeichnet dass** bei den zu behandelnden Personen der Nüchternblutzuckerwert 110 mg Glucose pro dl Plasma überschreitet.

9. Pharmazeutische Zusammensetzung zur Verwendung gemäß den Ansprüchen 1, 2 und 4, **dadurch gekennzeichnet dass** bei den zu behandelnden Personen der systolische Blutdruck einen Wert von 130 mm Hg und der diastolische Blutdruck einen Wert von 80 mm Hg übersteigt.

10. Pharmazeutische Zusammensetzung zur Verwendung gemäß der Ansprüche 1-9, **dadurch gekennzeichnet dass** bei den zu behandelnden Personen das Verhältnis von Taillienumfang zu Hüftumfang bei Frauen den Wert 0,8 und bei Männern den Wert 1 überschreitet.

## Claims

1. Pharmaceutical composition comprising an angiotensin II receptor antagonist selected from irbesartan and telmisartan or a salt thereof for use in a method for the treatment of individuals in whom Type 2 diabetes mellitus has been diagnosed or suspicion of prediabetes exists or for the prevention of diabetes.

2. Pharmaceutical composition for use according to claim 1, **characterised in that** the fasting blood sugar value exceeds 125 mg of glucose per d1 of plasma in the individuals to be treated.

3. Pharmaceutical composition for use according to claim 1, **characterised in that** the fasting blood sugar value is 110-125 mg of glucose per dl of plasma in the individuals to be treated.

4. Pharmaceutical composition for use according to claim 1, **characterised in that** a blood sugar value above 200 mg of glucose per dl of plasma is measured in the individuals to be treated 2 hours after fasting intake of 75 g of glucose.

5. Pharmaceutical composition for use according to claim 1, **characterised in that** a blood sugar value of 140-200 mg of glucose per dl of plasma is measured in the individuals to be treated 2 hours after fasting intake of 75 g of glucose.

6. Pharmaceutical composition for use according to claim 1, **characterised in that** the blood value for triglycerides exceeds 150 mg/dl in the individuals to be treated.

7. Pharmaceutical composition for use according to claim 6, **characterised in that** the blood value for HDL in women is less than 40 mg per dl of plasma, in men is less than 50 mg per dl of plasma in the individuals to be treated.

8. Pharmaceutical composition for use according to claims 6 and 7, **characterised in that** the fasting blood sugar value exceeds 110 mg of glucose per dl of plasma in the individuals to be treated.

9. Pharmaceutical composition for use according to claims 1, 2 and 4, **characterised in that** the systolic blood pressure exceeds a value of 130 mm Hg and the diastolic blood pressure exceeds a value of 80 mm Hg in the individuals to be treated.

10. Pharmaceutical composition for use according to claims 1-9, **characterised in that** the ratio of waist circumference to hip circumference in women exceeds the value 0.8 and in men exceeds the value 1 in the individuals to be treated.

## Revendications

1. Composition pharmaceutique comprenant un antagoniste du récepteur de l'angiotensine II choisi parmi l'irbésartan et le telmisartan ou un sel de ceux-ci pour l'utilisation dans un procédé pour le traitement de personnes chez qui un *diabetes mellitus* de type 2 a été diagnostiqué ou chez qui il existe une suspicion de prédiabète ou bien pour la prévention du diabète.

2. Composition pharmaceutique pour l'utilisation selon la revendication 1, **caractérisée en ce que** la glycémie à jeun dépasse 125 mg de glucose par dl de plasma chez les personnes à traiter.

3. Composition pharmaceutique pour l'utilisation selon la revendication 1, **caractérisée en ce que** la glycémie à jeun atteint 110 à 125 mg de glucose par dl de plasma chez les personnes à traiter.

4. Composition pharmaceutique pour l'utilisation selon la revendication 1, **caractérisée en ce que**, 2 heures après la prise à jeun de 75 g de glucose, une glycémie de plus que 200 mg de glucose par dl de plasma est mesurée chez les personnes à traiter.

5. Composition pharmaceutique pour l'utilisation selon la revendication 1, **caractérisée en ce que**, 2 heures après la prise à jeun de 75 g de glucose, une glycémie de 140-200 mg de glucose par dl de plasma est mesurée chez les personnes à traiter.

6. Composition pharmaceutique pour l'utilisation selon la revendication 1, **caractérisée en ce que** le taux sanguin de triglycérides dépasse 150 mg/dl chez les personnes à traiter.

7. Composition pharmaceutique pour l'utilisation selon la revendication 6, **caractérisée en ce que** le taux sanguin de HDL pour les femmes est inférieur à 40 mg par dl de plasma, le taux sanguin de HDL pour les hommes est inférieur à 50 mg par dl de plasma chez les personnes à traiter.

8. Composition pharmaceutique pour l'utilisation selon les revendications 6 et 7, **caractérisée en ce que** la glycémie à jeun dépasse 110 mg de glucose par dl de plasma chez les personnes à traiter.

9. Composition pharmaceutique pour l'utilisation selon les revendications 1, 2, et 4, **caractérisée en ce que** la pression sanguine systolique dépasse une valeur de 130 mm de Hg et la pression sanguine diastolique dépasse une valeur de 80 mm de Hg chez les personnes à traiter.

10. Composition pharmaceutique pour l'utilisation selon les revendications 1 à 9, **caractérisée en ce que** le rapport entre le tour de taille et le tour de hanche pour les femmes dépasse la valeur de 0,8 et pour les hommes la valeur de 1 chez les personnes à traiter.
